# EUROPEAN PATENT APPLICATION

(11) **EP 2 100 606 A1**
(43) Date of publication of application: **16.09.2009**
(21) Application number: 07850163.2
(22) Date of filing: 06.12.2007
(51) Int. Cl.: A61K 31/4365, A61K 9/30, A61K 9/36, A61K 47/32, A61K 47/36, A61K 47/38, A61P 7/02

(54) **FILM-COATED PREPARATION HAVING IMPROVED STABILITY**

(30) Priority: 07.12.2006 JP 2006330371
(71) Applicant: Daiichi Sankyo Company, Limited, Chuo-ku Tokyo 103-8426 (JP); Ube Industries, Ltd., Ube-shi Yamaguchi 755-8633 (JP)
(72) Inventor: WATANABE, Tomoyuki, Kanagawa 254-0014 (JP); MAEDA, Kazuko, Kanagawa 254-0014 (JP)
(74) Representative: Fairbairn, Angus Chisholm
(86) International application number: PCT/JP2007/073547
(87) International publication number: WO 2008/069262

(57) **Abstract**

Disclosed is a pharmaceutical composition containing a compound represented by the general formula (I) below, while having improved storage stability. Specifically disclosed is a film-coated preparation having excellent storage stability, which contains a compound represented by the general formula (I) below or a pharmacologically acceptable salt thereof, while having a film layer containing one or more film coating base agents selected from polyvinyl alcohol, sodium carboxymethyl cellulose and pullulan.

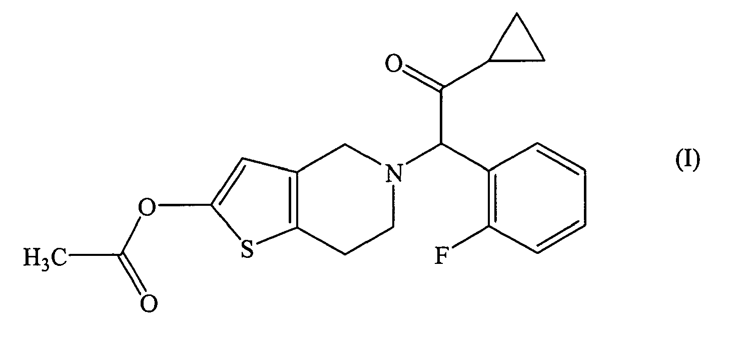

## Description

### [TECHNICAL FIELD]

The present invention relates to a film-coated preparation having excellent storage stability, which contains in its formulation a compound represented by the following general formula (I): 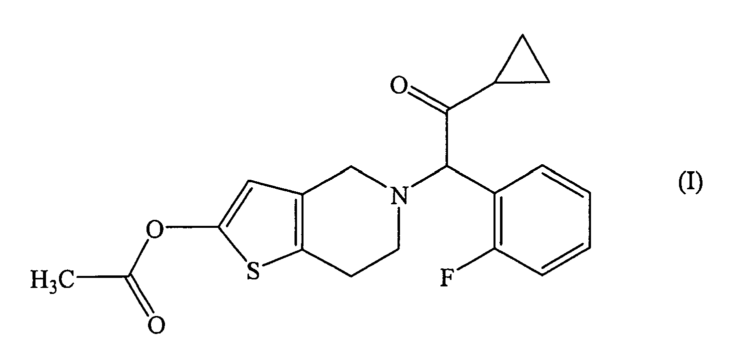 or a pharmacologically acceptable salt thereof, and which contains in its film layer one or more film coating base agents selected from polyvinyl alcohol, sodium carboxymethyl cellulose and pullulan.

### [BACKGROUND ART]

The compound represented by the aforementioned general formula (I) or a pharmacologically acceptable salt thereof is known as a compound having platelet aggregation inhibition activity (Patent Document 1 or 2).

Patent Documents 2, 3, 4 and 5 exemplify various kinds of additives that may be used in preparations containing the compound represented by the aforementioned general formula (I) or a pharmacologically acceptable salt thereof, and there is a line which mentions pullulan as a filler. However, there is no particular description with respect to polyvinyl alcohol and sodium carboxymethyl cellulose as such.

Further, Patent Documents 2, 4 and 5 merely describe that coatings may be provided if necessary, and polyvinyl alcohol, sodium carboxymethyl cellulose and pullulan are not specifically used in preparation examples. In addition, the aforementioned Patent Documents neither disclose nor teach that the stability of a preparation which contains the compound represented by the aforementioned general formula (I) or a pharmacologically acceptable salt thereof, can be improved by providing to the preparation a coating which contains polyvinyl alcohol, sodium carboxymethyl cellulose or pullulan as a film coating base agent.
[Patent Document 1] Japanese Patent Application (Kokai) No. Hei 6-41139
[Patent Document 2] Japanese Patent Application (Kokai) No. 2002-145883
[Patent Document 3] Japanese Patent Application (Kokai) No. Hei 10-310586
[Patent Document 4] Japanese Patent Application (Kokai) No. 2003-246735
[Patent Document 5] Japanese Patent Application (Kokai) No. 2004-51639

### [DISCLOSURE OF THE INVENTION]

### [PROBLEMS TO BE SOLVED BY THE INVENTION]

An object of the present invention is to provide a pharmaceutical composition having excellent storage stability, which contains the compound represented by the aforementioned general formula (I) or a pharmacologically acceptable salt thereof.

### [MEANS FOR SOLVING THE PROBLEMS]

As a result of conducting extensive studies to solve the aforementioned problem, the inventors of the present invention found that a film-coated preparation which contains the compound represented by the aforementioned general formula (I) or a pharmacologically acceptable salt thereof can have excellent storage stability by including a particular film coating base agent in the film layer, thereby leading to completion of the present invention.

The present invention provides a film-coated preparation which contains in its formulation the compound represented by the aforementioned general formula (I) or a pharmacologically acceptable salt thereof, and which contains in its film layer one or more film coating base agents selected from polyvinyl alcohol, sodium carboxymethyl cellulose and pullulan (particularly a film-coated preparation for prophylaxis or treatment of thrombosis or embolism), use of the compound represented by the aforementioned general formula (I) or a pharmacologically acceptable salt thereof for the production of the film-coated preparation (particularly the film-coated preparation for prophylaxis or treatment of thrombosis or embolism), and a prophylaxis or treatment strategy for a disease (particularly thrombosis or embolism) in which the film-coated preparation containing an effective amount of the compound represented by the aforementioned general formula (I) or a pharmacologically acceptable salt thereof is administered to a warm-blooded animal (particularly a human).

That is, the present invention is:
(1) a film-coated preparation which contains in its formulation the compound represented by the aforementioned general formula (I) or a pharmacologically acceptable salt thereof, and which contains in its film layer one or more film coating base agents selected from polyvinyl alcohol, sodium carboxymethyl cellulose and pullulan, preferably,
(2) the film-coated preparation according to (1), wherein the compound represented by the general formula (I) or a pharmacologically acceptable salt thereof is a compound represented by the following formula (Ia): 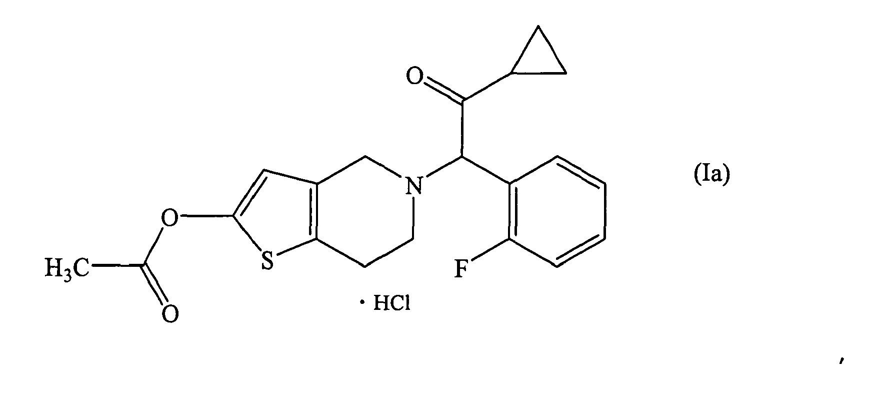
(3) the film-coated preparation according to (1) or (2), wherein the film coating base agent is polyvinyl alcohol,
(4) the film-coated preparation according to (1) or (2), wherein the film coating base agent is sodium carboxymethyl cellulose,
(5) the film-coated preparation according to (1) or (2), wherein the film coating base agent is pullulan, or
(6) the film-coated preparation according to any one of (1) to (5), wherein the preparation is in the form of tablets.

### [EFFECT OF THE INVENTION]

According to the present invention, a film-coated preparation having excellent storage stability can be provided, which contains in its formulation the compound represented by the aforementioned general formula (I) or a pharmacologically acceptable salt thereof, and which contains in its film layer one or more film coating base agents selected from polyvinyl alcohol, sodium carboxymethyl cellulose and pullulan.

The film-coated preparation of the present invention is, for example, useful for the treatment and/or prophylaxis of thrombosis or embolism (preferably thrombosis) and the like (preferably is a drug for the treatment and/or prophylaxis of thrombosis).

### [BEST MODE FOR CARRYING OUT THE INVENTION]

The compound represented by the aforementioned general formula (I) or a pharmacologically acceptable salt thereof, which is the active ingredient of the film-coated preparation of the present invention, that is, 2-acetoxy-5-(α-cyclopropylcarbonyl-2-fluorobenzyl)-4,5,6,7-tetrahydrothieno[3,2-c]pyridine or a pharmacologically acceptable salt thereof, is a publicly known compound, and can be produced in accordance with the methods described in Japanese Patent Application (Kokai) No. Hei 6-41139 or Japanese Patent Application (Kokai) No. 2002-145883.

As the "pharmacologically acceptable salt thereof" of the present invention, there may be mentioned for example, hydrohalide salts such as hydrofluoride, hydrochloride, hydrobromide or hydroiodide; inorganic acid salts such as nitrates, perchloric acid salt, sulfates or phosphates; lower-alkyl sulfonic acid salts such as methanesulfonate, trifluoromethanesulfonate or ethanesulfonate; aryl sulfonic acid salts such as benzenesulfonate or p-toluenesulfonate; organic acid salts such as acetate, malate, fumarate, succinate, citrate, ascorbate, tartrate, oxalate or maleate; or amino acid salts such as glycine salt, lysine salt, arginine salt, ornithine salt, glutamic acid salt or aspartic acid salt, preferably hydrohalide salts or organic acid salts, more preferably hydrochloride or maleate, and most preferably hydrochloride.

The film coating base agent of the present invention is one or more selected from polyvinyl alcohol, sodium carboxymethyl cellulose and pullulan, preferably polyvinyl alcohol or sodium carboxymethyl cellulose. With respect to the amount of the film coating base agent contained in the film layer, the lower limit is usually 20% (w/w), preferably 30% (w/w) and more preferably 40% (w/w), and the upper limit is usually 100% (w/w), preferably 90% (w/w) and more preferably 70% (w/w).

Other coating base agents may be formulated into the film layer if necessary. The type of such coating base agents is not particularly limited, and a person skilled in the art can select them appropriately. As such coating base agents, there may be mentioned for example, polyvinylpyrrolidone (PVP), methyl cellulose, ethyl cellulose, hydroxypropyl cellulose (HPC), hydroxypropylmethyl cellulose (HPMC), dextrin, maltodextrin, lactose, D-mannitol, polyvinyl alcohol polymer, methacrylic acid copolymer, aminoalkylmethacrylate copolymer and ethyl acrylate• methyl methacrylate copolymer.

The film-coated preparation of the present invention may further contain additives such as appropriate pharmacologically acceptable fillers, lubricants, binders, emulsifiers, stabilizers, corrigents and/or diluents.

As the "fillers" used, there may be mentioned for example, organic fillers including sugar derivatives such as lactose, sucrose, glucose, mannitol or sorbitol; starch derivatives such as corn starch, potato starch, α-starch or dextrin; cellulose derivatives such as crystalline cellulose; gum Arabic; or dextran: or inorganic fillers including silicate derivatives such as light anhydrous silicic acid, synthetic aluminum silicate, calcium silicate or magnesium metasilicate aluminate; phosphates such as calcium hydrogen phosphate; carbonates such as calcium carbonate; or sulfates such as calcium sulfate. Of these, one or more fillers selected from cellulose derivatives and sugar derivatives are preferably used, one or more fillers selected from lactose, mannitol and crystalline cellulose are more preferably used, and one or more fillers selected from lactose and/or crystalline cellulose are most preferably used.

As the "lubricants" used, there may be mentioned for example, stearic acid; stearic acid metal salts such as calcium stearate or magnesium stearate; talc; colloidal silica; waxes such as beeswax or spermaceti; boric acid; adipic acid; sulfates such as sodium sulfate; glycol; fumaric acid; sodium stearyl fumarate; sucrose fatty acid esters; sodium benzoate; D,L-leucine; lauryl sulfates such as sodium lauryl sulfate or magnesium lauryl sulfate; silicates such as silicic anhydride or hydrated silicate; or the aforementioned starch derivatives. Of these, stearic acid metal salts are preferably used.

As the "binders" used, there may be mentioned for example, hydroxypropyl cellulose, hydroxypropylmethyl cellulose, polyvinylpyrrolidone, polyethylene glycol, or the same compounds as those mentioned for the fillers. Of these, hydroxypropyl cellulose or hydroxypropylmethyl cellulose is preferably used.

As the "emulsifiers" used, there may be mentioned for example, colloidal clays such as bentonite or beegum; metal hydroxides such as magnesium hydroxide or aluminum hydroxide; anionic surfactants such as sodium lauryl sulfate or calcium stearate; cationic surfactants such as benzalkonium chloride; or nonionic surfactants such as polyoxyethylene alkyl ether, polyoxyethylene sorbitan fatty acid ester or sucrose fatty acid ester.

As the "stabilizers" used, there may be mentioned for example, para-oxybenzoic acid esters such as methyl paraben or propyl paraben; alcohols such as chlorobutanol, benzyl alcohol or phenyl ethyl alcohol; benzalkonium chloride; phenols such as phenol or cresol; thimerosal; dehydroacetic acid; or sorbic acid.

As the "corrigents" used, there may be mentioned for example, sweeteners such as sodium saccharin or aspartame; acidulants such as citric acid, malic acid or tartaric acid; or flavorings such as menthol, lemon or orange.

Although there is no particular limitation as regards the amount of the compound represented by the aforementioned general formula (I) or a pharmacologically acceptable salt thereof formulated in the entirety of the film-coated preparation, it is preferable to formulate 1.0 to 30.0 % by weight (preferably 1.3 to 20.0 % by weight) with respect to the total weight of the film-coated preparation.

Although there is no particular limitation as regards the amount of additives formulated in the entirety of the film-coated preparation, it is preferable to formulate 10.0 to 93.5 % by weight (preferably 44.0 to 90.0 % by weight) of fillers, 0.5 to 5.0 % by weight (preferably 0.5 to 3.0 % by weight) of lubricants, and 0.0 to 15.0 % by weight (preferably 2.5 to 10.0 % by weight) of binders with respect to the total weight of the film-coated preparation.

As regards film-coated preparations of the present invention, there may be mentioned for example, solid dosage forms such as tablets, capsules, powders, fine granules, granules or troches, preferably tablets.

As regards production methods for the pharmaceutical preparation of the present invention, it can be produced by using a general method described in publications such as "Powder Technology and Pharmaceutical Process (D. Chulia et al., Elsevier Science Pub Co (December 1, 1993))". In particular, a dry method (for example, a dry granulation method or a direct compression method, preferably a direct compression method) is preferable.

The "direct compression method" is a method in which raw material powders are directly subjected to compression-molding to produce a preparation.

The "dry granulation method" is a method in which a preparation is produced using granules prepared by crushing and dividing by an appropriate method a compression-molded slug or sheet of raw material powders. These methods are described in publications such as "The Theory and Practice of Industrial Pharmacy (Third Edition) (Leon Lachman et al.: LEA & FEBIGER 1986)" and "Pharmaceutical Dosage Forms: Tablets volume 1 (Second Edition) (Herbert A. Lieberman et al.: MARCEL DEKKER INC. 1989)".

The "granulation" used here means an operation of forming granules having an almost uniform shape and size from a raw material in the form of powders, mass, solution, or molten liquid, and examples include granulation for forming a final product such as granules, powders or fine granules, and granulation for forming an intermediate product for the production of a tablet or a capsule.

The compression-molding process is a process in which a mass product of raw material powder is formed by applying pressure to the raw material powder using mechanical force, and examples of apparatuses used include rotary tableting machines (manufactured by Kikusui Seisakusho Ltd., Hata Iron Works Co., Ltd., Sugawara Seiki Co., Ltd. and the like), and dry granulators such as a roller compactor, a roll granulator, and a Chilsonator (manufactured by Freund Corporation, Turbo Kogyo Co., Ltd., Kurimoto, Ltd., Matsubo Corporation, Nippon Granulator Co., Ltd., Fuji Paudal Co., Ltd. and the like).

The crushing and dividing process is a process in which the mass product formed in the compression-molding process is crushed by means of a knife or cutter into an appropriate size, and examples of apparatuses used include mills and particle size selectors such as a power mill, Fitzmill, Fiore, and Co-mill (manufactured by Fuji Paudal Co., Ltd., Tokuju Corporation, Powrex Corporation and the like).

The thus obtained granulated product is subjected to particle size regulation so as to have a desired particle diameter, and then a preparation in the form of powders, fine granules or granules is produced. These preparations can also be produced as capsules by packing them in a capsule, or can be produced as tablets by further adding disintegrants and/or lubricants if necessary and subjecting them to compression-molding by a tableting machine or the like. The operations of mixing and granulation are both widely used in the field of formulation techniques, and those skilled in the art can carry them out appropriately.

The film-coated preparation of the present invention may, if necessary, further contain in the formulation one or more additives such as plasticizers, fillers, lubricants, masking agents, colorants and antiseptics in amounts generally used.

The plasticizers which may be used in the present invention are not particularly limited, and a person skilled in the art can select them appropriately. As for such plasticizers, there may be mentioned for example, propylene glycol, polyethylene glycol, polypropylene glycol, glycerin and sorbitol, glycerin triacetate, diethyl phthalate and triethyl citrate, lauric acid, sucrose, dextrose, sorbitol, triacetin, acetyl triethyl citrate, triethyl citrate, tributyl citrate or acetyl tributyl citrate.

As the masking agents which may be used in the present invention, there may be mentioned for example, titanium oxide.

As the colorants which may be used in the present invention, there may be mentioned for example, titanium oxide, iron oxide, red ferric oxide, yellow ferric oxide or yellow No. 5 aluminum lake talc.

As the antiseptics which may be used in the present invention, there may be mentioned for example, paraben.

As regards production methods for the film-coated preparation of the present invention, there may be used a general method described in publications such as "Powder Technology and Pharmaceutical Process (D. Chulia et al., Elsevier Science Pub Co (December 1, 1993))", and there is no particular limitation.

The film-coated preparation which is prepared by the present method can be obtained by spraying a film-coating solution containing one or more film coating base agents selected from polyvinyl alcohol, sodium carboxymethyl cellulose and pullulan, to an object to be coated such as a tablet or drug bulk. The object to be coated may be provided with a sub-coating if necessary. The film-coating solution can be obtained by suspending or dissolving in water one or more film coating base agents selected from polyvinyl alcohol, sodium carboxymethyl cellulose and pullulan, and the aforementioned additives which are formulated if necessary. Spraying of the film-coating solution can be conducted by publicly known methods such as by using a commercially available film-coating machine. Film-coating is conducted under conditions in which the lower limit is preferably 3% (w/w), more preferably 4.5% (w/w), and particularly preferably 6% (w/w), and the upper limit is preferably 50% (w/w) and more preferably 20% (w/w), with respect to the uncoated tablet. Conditions under which usual film-coated preparations are produced can be adopted as the production conditions. The film-coated preparation of the present invention thus obtained can be administered in a similar manner to usual preparations.

The dosage amount of the compound represented by the aforementioned general formula (I) or a pharmacologically acceptable salt thereof, which is an active ingredient of the film-coated preparation of the present invention, may vary depending on various conditions such as the activity of the drug, symptoms, age or body weight of a patient. The daily dosage amount for an adult human has a lower limit of 0.01 mg (preferably 1 mg) and an upper limit of 200 mg (preferably 100 mg) in the case of oral administration.

### [Examples]

The present invention will be described in more detail with reference to the Examples and Test Examples; however, the present invention shall not be limited to these.

Here, "Compound A" used in the Examples is the compound represented by the aforementioned formula (Ia).

### Example 1

Compound A (54.9 g), hydroxypropyl cellulose (150.0 g), low-substituted hydroxypropyl cellulose (400.0 g), lactose (1175.0 g) and crystalline cellulose (200.0 g) were mixed using a high intensity mixer for 3 minutes, followed by addition of magnesium stearate (20.0 g), and the mixture was mixed again using the high intensity mixer to give a mixed powder.

The mixed powder obtained was compressed using a rotary type compression machine with a tableting pressure of 6.9 kN so that the tablet mass became approximately 100 mg. The uncoated tablet obtained was subjected to film-coating in a pan-coating machine, by spraying a coating solution consisting of 5% (w/w) sodium carboxymethyl cellulose and water, to give a tablet containing the test compound. Results of stability testing conducted for the obtained tablet are shown in Table 1.

### Example 2

Compound A (54.9 g), hydroxypropyl cellulose (150.0 g), low-substituted hydroxypropyl cellulose (400.0 g), lactose (1175.0 g) and crystalline cellulose (200.0 g) were mixed using a high intensity mixer for 3 minutes, followed by addition of magnesium stearate (20.0 g), and the mixture was mixed again using the high intensity mixer to give a mixed powder.

The mixed powder obtained was compressed using a rotary type tableting machine with a tableting pressure of 6.9 kN so that the tablet mass became approximately 100 mg. The uncoated tablet obtained was subjected to film-coating in a pan-coating machine, by spraying a coating solution consisting of OPADRY AMB 31W48994 (manufactured by Colorcon (Japan) Limited) having polyvinyl alcohol as the principal component and water, to give a tablet containing the test compound. Results of stability testing conducted on the obtained tablet are shown in Table 1.

### Comparative Example 1

Compound A (24.7 g), hydroxypropyl cellulose (67.5 g), low-substituted hydroxypropyl cellulose (180.0 g), lactose (528.8 g) and crystalline cellulose (90.0 g) were mixed using a high intensity mixer for 3 minutes, followed by addition of magnesium stearate (9.0 g), and the mixture was mixed again using the high intensity mixer to give a mixed powder.

The mixed powder obtained was compressed using a rotary type tableting machine with a tableting pressure of 6.9 kN so that the tablet mass became approximately 100 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Results of stability testing conducted on the obtained tablet are shown in Table 1.

### Test Example 1 Stability Test

Tablets obtained in Examples 1 and 2 and Comparative Example 1 were each placed in an amber glass bottle, and were left to stand at 60°C under sealed conditions. After 3 weeks, the content of the active ingredient (the compound represented by the general formula (I)) in the test tablets was measured by high performance liquid chromatography. The measuring conditions for the high performance liquid chromatography were as follows.
Column: L-column ODS (4.6 mmID x 150 mm, manufactured by Chemicals Evaluation and Research Institute, Japan)
Mobile Phase: 0.01 mol/L phosphate buffer solution (pH 2.8)/acetonitrile mixture = 65/35 (v/v)
Column Temperature: constant temperature around 40°C
Wavelength detected: 260 nm

**Table 1**

| | Example 1 | Example 2 | Comparative Example 1 |
|---|---|---|---|
| Amount of active ingredient remaining after 3 weeks at 60°C (%) | 94 | 91 | 78 |

### [INDUSTRIAL APPLICABILITY]

According to the present invention, a film-coated preparation having excellent storage stability, which contains the compound represented by the aforementioned general formula (I) or a pharmacologically acceptable salt thereof, and which contains in its film layer one or more film coating base agents selected from polyvinyl alcohol, sodium carboxymethyl cellulose and pullulan, can be obtained.

## Claims

1. A film-coated preparation which contains in its formulation the compound represented by the following general formula (I): 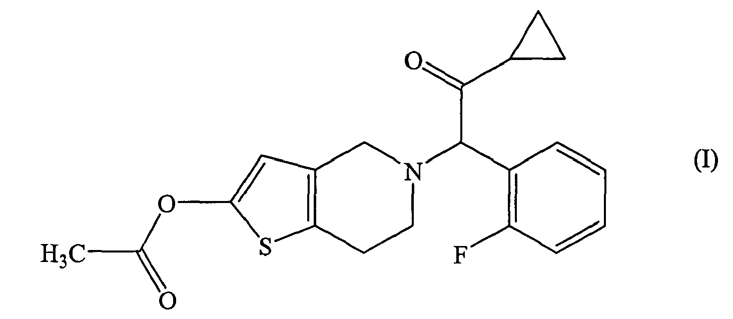 or a pharmacologically acceptable salt thereof, and which contains in its film layer one or more film coating base agents selected from polyvinyl alcohol, sodium carboxymethyl cellulose and pullulan.

2. The film-coated preparation according to claim 1, wherein the compound represented by the general formula (I) or a pharmacologically acceptable salt thereof is a compound represented by the following formula (Ia):

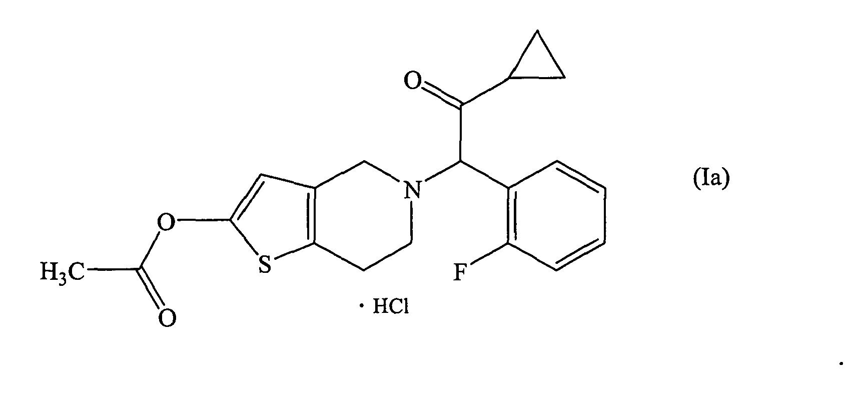

3. The film-coated preparation according to claim 1 or 2, wherein the film coating base agent is polyvinyl alcohol.

4. The film-coated preparation according to claim 1 or 2, wherein the film coating base agent is sodium carboxymethyl cellulose.

5. The film-coated preparation according to claim 1 or 2, wherein the film coating base agent is pullulan.

6. The film-coated preparation according to any one of claims 1 to 5, wherein the preparation is in the form of tablets.
